(19) Europäisches Patentamt
European Patent Office
Office européen des brevets

(11) **EP 4 413 921 A1**

(12) **EUROPEAN PATENT APPLICATION**
published in accordance with Art. 153(4) EPC

(43) Date of publication:
**14.08.2024 Bulletin 2024/33**

(21) Application number: **22878613.3**

(22) Date of filing: **07.10.2022**

(51) International Patent Classification (IPC):
**A61B 5/055** (2006.01)    **G06T 7/00** (2017.01)

(52) Cooperative Patent Classification (CPC):
**A61B 5/055; G06T 7/00**

(86) International application number:
**PCT/JP2022/037568**

(87) International publication number:
**WO 2023/058746 (13.04.2023 Gazette 2023/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.10.2021    JP 2021166163**

(71) Applicant: **Sapporo Medical University**
**Sapporo-shi, Hokkaido 060-8556 (JP)**

(72) Inventor: **AKIYAMA, Yukinori**
**Sapporo-shi, Hokkaido 060-8556 (JP)**

(74) Representative: **v. Bezold & Partner Patentanwälte - PartG mbB**
**Ridlerstraße 57**
**80339 München (DE)**

(54) **IMAGE DIAGNOSIS APPARATUS, METHOD FOR OPERATING IMAGE DIAGNOSIS APPARATUS, AND PROGRAM**

(57)    An image diagnosis apparatus (100) includes an acquirer (152) to acquire a tomographic image including a region to be diagnosed of a subject and a drawer (153) to draw, based on a tomographic image acquired by the acquirer (152), a labeled image including the region to be diagnosed, the labeled image being partitioned according to classes indicating a lesion area, a normal tissue area, and a background area. The normal tissue area may be partitioned into a cavity area, a soft tissue area, and a bone area. The lesion area is a tumor area inside the brain, the tomographic image is an image of a cross section obtained by slicing the brain of the subject in the transverse plane direction at a plurality of points, and the drawer (153) may draw a labeled image corresponding to each tomographic image acquired by the acquirer (152).

FIG.2A

## Description

Technical Field

The present disclosure relates to an image diagnosis apparatus, a method for operating the image diagnosis apparatus, and a program.

Background Art

In order to assist diagnosis of a tumor performed by a doctor, an effort to perform machine learning, using images acquired from a lot of patients and perform image diagnosis with respect to each patient, using a training model obtained by the machine learning has been made. For example, in Patent Literature 1, an automatic brain tumor diagnosis method for determining what type of tissue, specifically which one of glioblastoma and meningioma, a brain tumor in an image is, using a trained model obtained by machine learning is disclosed.

Citation List

Patent Literature

Patent Literature 1: Unexamined Japanese Patent Application Publication No. 2018-515164

Summary of Invention

Technical Problem

The method disclosed in Patent Literature 1 only determines what type of brain tumor a brain tumor the existence of which has been known, and, for example, when a contrast effect for a brain tumor is small, when multiple brain tumors have developed, or when a brain tumor is close to a blood vessel or a brain surface and adjacent to a normal structure, it is difficult in the first place to detect existence itself of a brain tumor. Image diagnosis of a brain tumor is a heavy burden for an inexperienced diagnostician or a doctor having a different area of expertise, and practical implementation of a method for assisting simple and accurate diagnosis of existence or nonexistence of a brain tumor is expected. Such a problem exists not only in a case where existence or nonexistence of a brain tumor is diagnosed but also in a case where existence or nonexistence of another lesion is diagnosed.

The present disclosure has been made in consideration of the above-described circumstances, and an objective of the present disclosure is to provide an image diagnosis apparatus, a method for operating the image diagnosis apparatus, and a program that assists simple and accurate diagnosis of existence or nonexistence of a lesion in a region to be diagnosed.

Solution to Problem

In order to achieve the above-described objective, an image diagnosis apparatus according to a first aspect of the present disclosure includes:

> an acquirer to acquire a tomographic image including a region to be diagnosed of a subject; and
> a drawer to draw, based on a tomographic image acquired by the acquirer, a labeled image including the region to be diagnosed, the labeled image being partitioned according to classes each of which indicates one of a lesion area, a normal tissue area, and a background area.

The normal tissue area may be partitioned into a cavity area, a soft tissue area, and a bone area.

The lesion area may be a tumor area in a brain,

> the tomographic image may be an image of a cross section of a brain of a subject, the cross section being obtained by slicing the brain in a transverse plane direction at a plurality of points, and
> the drawer may draw a labeled image corresponding to each tomographic image acquired by the acquirer.

The tumor area may be an area where a metastatic brain tumor has developed.

The labeled image may have a unique pixel value with respect to each class, and
the drawer may estimate, based on a model that, with respect to input of a pixel value of each pixel in a tomographic image, outputs a pixel value of each pixel in a labeled image, a pixel value of each pixel in a labeled image from a tomographic image acquired by the acquirer.

The image diagnosis apparatus may further include a trainer to generate the model by machine learning, and
the drawer may estimate, based on the model generated by the trainer, a pixel value of each pixel in a labeled image from a pixel value of each pixel in a tomographic image acquired by the acquirer.

The trainer may generate the model, using training data that include a pixel value of each pixel in a tomographic image as input data and a pixel value of each pixel in a labeled image as output data, the labeled image being generated based on the tomographic image and having a weight of each class adjusted based on the number of counted pixels of the class.

The trainer may generate the model, using training data generated based on a plurality of tomographic images that has cross sections obtained by slicing a brain of each of a plurality of subjects in a transverse plane direction at a plurality of points.

The image diagnosis apparatus may further include an outputter to color-code a labeled image with respect to each class, the labeled image being drawn by the drawer.

In order to achieve the above-described objective, a method for operating an image diagnosis apparatus according to a second aspect of the present disclosure is

a method for operating the image diagnosis apparatus including an acquirer and a drawer, and the method includes:

acquiring, by the acquirer, a tomographic image including a region to be diagnosed of a subject; and

drawing, by the drawer, based on a tomographic image acquired by the acquirer, a labeled image including the region to be diagnosed, the labeled image being partitioned according to classes each of which indicates one of a lesion area, a normal tissue area, and a background area.

In order to achieve the above-described objective, a program according to a third aspect of the present disclosure causes

a computer to function as:

acquisition means for acquiring a tomographic image including a region to be diagnosed of a subject; and

drawing means for drawing, based on a tomographic image acquired by the acquisition means, a labeled image including the region to be diagnosed, the labeled image being partitioned according to classes each of which indicates one of a lesion area, a normal tissue area, and a background area.

Advantageous Effects of Invention

According to the present disclosure, an image diagnosis apparatus, a method for operating the image diagnosis apparatus, and a program that assist simple and accurate diagnosis of existence or nonexistence of a lesion in a region to be diagnosed can be provided.

Brief Description of Drawings

FIG. 1 is a schematic diagram illustrating a configuration of an image diagnosis system according to an embodiment of the present disclosure;
FIG. 2A is a block diagram illustrating a hardware configuration of an image diagnosis apparatus according to the embodiment of the present disclosure;
FIG. 2B is a diagram illustrating an example of a data table of an image data storage according to the embodiment of the present disclosure;
FIG. 3 is a diagram for a description of a set of training data according to the embodiment of the present disclosure;
FIG. 4 is a conceptual diagram of a neural network that is used in drawing of a labeled image performed by the image diagnosis apparatus according to the embodiment of the present disclosure;
FIG. 5 is a diagram illustrating a process for optimizing weights between respective layers in the neural network performed by the image diagnosis apparatus according to the embodiment of the present disclosure;
FIG. 6 is a flowchart illustrating a flow of training processing according to the embodiment of the present disclosure;
FIG. 7 is a flowchart illustrating a flow of weighting coefficient optimization processing according to the embodiment of the present disclosure;
FIG. 8 is a flowchart illustrating a flow of diagnosis processing according to the embodiment of the present disclosure;
FIG. 9A is a graph illustrating the number of counted pixels of each label in a labeled image in an example;
FIG. 9B is a diagram illustrating an example of a data table indicating weights of respective labels in the example;
FIG. 10A is a diagram illustrating a manner in which a small single lesion is detected in the example;
FIG. 10B is a diagram illustrating a manner in which a lesion having a low pixel value is detected in the example;
FIG. 11A is a diagram illustrating a manner in which extremely small lesions are detected in the example; and
FIG. 11B is a diagram illustrating a manner in which multiple lesions are detected in the example.

Description of Embodiments

An image diagnosis apparatus, a method for operating the image diagnosis apparatus, and a program according to an embodiment of the present disclosure are described in detail below with reference to the drawings. In the respective drawings, the same or equivalent parts are designated by the same reference numerals. Although in the embodiment, description is made using as an example a case where a magnetic resonance imaging (MRI) image is used as a tomographic image, the tomographic image may be an image other than an MRI image, such as a computed tomography (CT) image, an X-ray tomographic image, and an ultrasonic tomographic image.

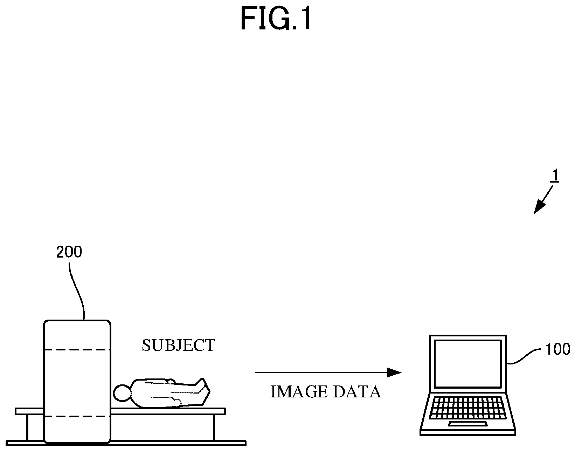

FIG. 1 is a schematic diagram illustrating a configuration of an image diagnosis system 1 according to the embodiment. The image diagnosis system 1 includes an image diagnosis apparatus 100 and an MRI apparatus 200. The MRI apparatus 200 is an example of an image capturing apparatus that, by capturing an image of the brain that is a region to be diagnosed of a subject, acquires a tomographic image including the brain of the subject. The image diagnosis apparatus 100 and the MRI apparatus 200 are interconnected in a communicable manner via a wired or wireless communication line, and the image diagnosis apparatus 100 receives image data transmitted from the MRI apparatus 200.

The image diagnosis apparatus 100 is an apparatus that, by applying image processing to an MRI image of the brain, diagnoses whether or not a brain tumor exists in the brain of the subject. The MRI image of the brain is an image of a cross section of the brain of the subject acquired by slicing the brain in the transverse plane direction. The MRI image of the brain is preferably one of the images that are acquired by slicing the brain of the same subject at a plurality of positions separated from each other in the longitudinal direction of the subject at, for example, a uniform pitch in the longitudinal direction of the subject. In addition, the MRI image is preferably a T1-weighted image (T1WI) that is captured while a Gadolinium-based contrast agent (Gd-based contrast agent) is administered to the subject.

Brain tumors to be diagnosed include both a primary brain tumor and a metastatic brain tumor. Primary brain tumors include a benign brain tumor, such as a meningioma, a pituitary adenoma, and a neurinoma, and a malignant brain tumor, such as a glioma and a central nervous system malignant lymphoma. Gliomas include a highly malignant glioblastoma. In addition, it is assumed that brain tumors include cerebrospinal fluid dissemination where tumor cells float in cerebrospinal fluid.

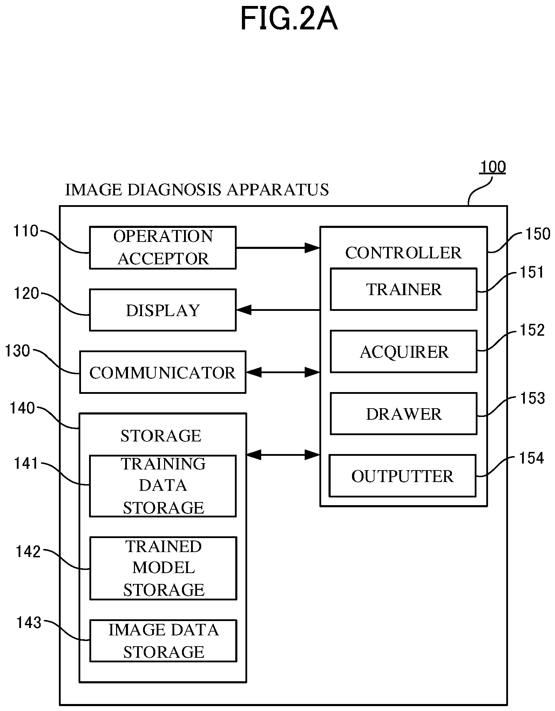

FIG. 2A is a block diagram illustrating a hardware configuration of the image diagnosis apparatus 100 according to the embodiment. The image diagnosis apparatus 100 is, for example, a general-purpose computer. The image diagnosis apparatus 100 includes an operation acceptor 110, a display 120, a communicator 130, a storage 140, and a controller 150. The respective units of the image diagnosis apparatus 100 are interconnected via an internal bus (not illustrated).

The operation acceptor 110 accepts an instruction from a user, such as a doctor, and supplies the controller 150 with an operation signal corresponding to an accepted operation. The operation acceptor 110 includes, for example, a mouse and a keyboard.

The display 120 displays various types of images to the user, based on image data supplied from the controller 150. The display 120 displays, for example, an MRI image of the subject that is captured by the MRI apparatus 200.

The operation acceptor 110 and the display 120 may be configured by a touch panel. The touch panel displays an operation screen for accepting predetermined operations, as well as supplying the controller 150 with an operation signal corresponding to a position at which a measurer performs a touch operation on the operation screen.

The communicator 130 is an interface capable of connecting to a communication network, such as the Internet. The communicator 130 receives, for example, image data relating to MRI images from the MRI apparatus 200.

The storage 140 includes, for example, a random access memory (RAM), a read only memory (ROM), a flash memory, and a hard disk. The storage 140 stores programs that are executed by the controller 150 and various types of data. In addition, the storage 140 also functions as a working memory to temporarily store various types of information and for the controller 150 to perform processing. Further, the storage 140 includes a training data storage 141, a trained model storage 142, and an image data storage 143.

The training data storage 141 stores training data that are used as teacher data in machine learning. The training data include a plurality of data sets, and each data set includes one MRI image acquired from the subject and one labeled image generated based on the MRI image by a person who is experienced in image diagnosis, such as a doctor. The labeled image is partitioned based on classes that are set on the MRI image. A class is a region to which a unique pixel value is assigned with respect to each piece of tissue of the subject.

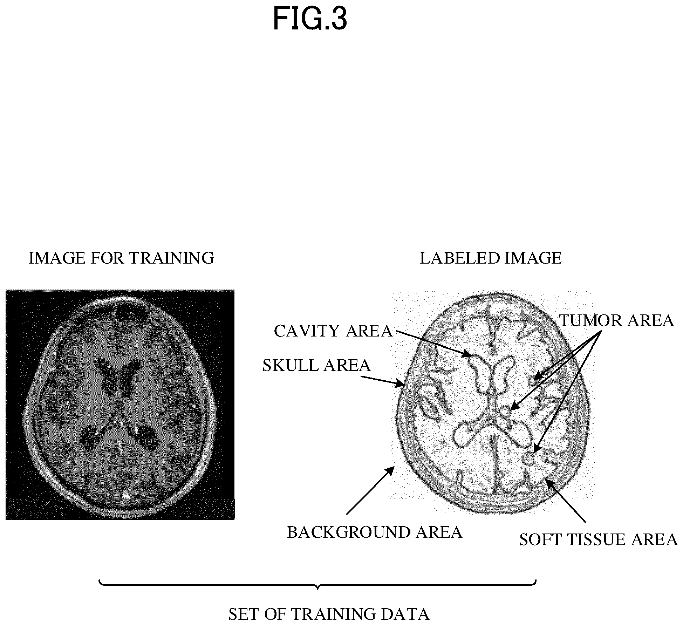

FIG. 3 is a diagram for a description of a set of training data according to the embodiment. In FIG. 3, to facilitate understanding, outlines of classes in the labeled image are illustrated in an emphasized manner. The MRI image and the labeled image are, for example, grayscale images represented by pixel values of 0 to 255 (256 gradations) and have the same number of pixels and aspect ratio. The labeled image is generated by segmenting all the pixels in the MRI image with respect to each class. Each class is classified into one of a tumor area, a cavity area, a soft tissue area, a skull area, and a background area, and to each pixel in the MRI image, a pixel value corresponding to one of the classes is assigned. The labeled image is generated by a person who is experienced in the image diagnosis assigning a gradation to each pixel with reference to the original MRI image, such as assigning a pixel value of A to a pixel since the pixel is equivalent to the background area and assigning a pixel value of B to another pixel since the pixel is equivalent to the skull area.

The tumor area is an area of a tumor that has developed in the brain and is an example of a lesion area. A plurality of tumor areas may exist according to the number of tumors that have developed. The tumor areas may include only a single type of tumor, such as a metastatic brain tumor, or may include different types of tumors. The cavity area is an area of a cavity that exists inside brain tissue or between the brain tissue and the skull, and the inside of the cavity area is filled with cerebrospinal fluid. The soft tissue area is normal tissue where no tumor has developed within the brain tissue. The pia mater, the arachnoid mater, and the dura mater adhered to the brain tissue are also included in the soft tissue area as long as no tumor has developed thereon. The skull area is an example of a bone area and includes not only the skull but also skin tissue covering the skull. The background area is an area other than the human body that exists around the skull area.

When a labeled image to be used as training data is generated, the pixel values of the respective classes in the labeled image are preferably set, using weighting of the classes. This is because there is a possibility that a class occupying a wide area within an image, such as the background area and the skull area, in other words, a class ranked higher in terms of the number of counted pixels, is biased and such a biased class has an adverse effect on a result of training processing. To avoid such a drawback, it is only required that the number of pixels of each class in the labeled image is counted, class weights are set based on the numbers of counted pixels of the respective labels, and the pixel values of the respective classes are adjusted based on the set class weights.

Specifically, the weight of a class having a large number of counted pixels is set to a small value, and the weight of a class having a small number of counted pixels is set to a large value. For example, the weight of a class in which the number of counted pixels is a median frequency value is set to 1, and the pixel values of the respective classes are adjusted by setting the weights of the classes in such a manner that the larger the number of counted pixels of the class is than the median frequency value, the smaller the weight is and the smaller the number of counted pixels of the class is than the median frequency value, the larger the weight is.

Returning to FIG. 2A, the trained model storage 142 stores a trained model that is generated by machine learning, based on the training data stored in the training data storage 141. The trained model is a model that, with respect to input of a pixel value of each pixel in the MRI image, outputs a pixel value of each pixel in the labeled image. In the generation of the trained model, for example, a neural network is used.

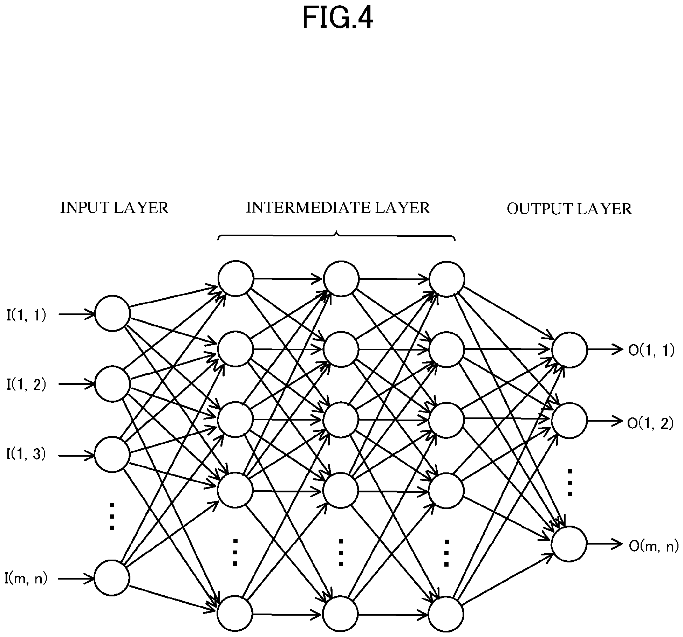

FIG. 4 is a conceptual diagram of the neural network that is used in drawing a labeled image by the image diagnosis apparatus 100 according to the present embodiment. The neural network includes an input layer to which input data are input, an output layer from which output data are output, and at least one intermediate layer that is arranged between the input layer and the output layer. Arrows between neurons in the respective layers represent relationships among parameters between the input layer and the output layer.

The input layer includes a plurality of neurons for input. The number of neurons for input in the input layer corresponds to the number of pieces of input data. The input data are pixel values of the respective pixels in the MRI image, and when it is assumed that the number of pixels in the MRI image is m pixels multiplied by n pixels, the input data can be denoted as $I(1, 1)$, $I(1,2)$, ... , $I(m, n)$. Each piece of input data input to a neuron for input in the input layer is input to respective intermediate neurons in the intermediate layers.

Each of the intermediate layers includes a plurality of intermediate neurons. Each neuron in the intermediate layers, when receiving input values I from respective neurons in a preceding stage, calculates a product I·W of the input values I and weighting coefficients W, calculates a sum of products I·W calculated with respect to each neuron in the preceding stage, and outputs an output value by substituting the sum of products I·W into an activation function. The activation function is a function that represents a nonlinear relationship between an input and an output at one neuron and is, for example, a sigmoid function, a max function, or a Gaussian function.

The output layer includes a plurality of neurons for output. The number of neurons for output corresponds to the number of pieces of output data. When receiving input values I from respective neurons in an intermediate layer at the rearmost position, each neuron in the output layer, as with each neuron in the intermediate layers, calculates a product I·W of the input values I and weighting coefficients W, calculates a sum of products I·W calculated with respect to each neuron in the preceding stage, and outputs an output value by substituting the sum of products I·W into an activation function. The output data are pixel values of the respective pixels in the labeled image, and when it is assumed that the number of pixels in the labeled image is m pixels multiplied by n pixels, the output data can be denoted as $O(1, 1)$, $O(1,2)$, ... , $O(m, n)$.

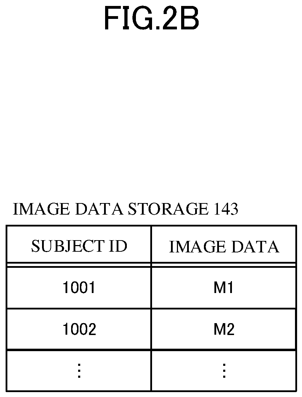

The image data storage 143 stores image data acquired from the MRI apparatus 200 in association with a subject identification (ID) assigned to each subject, as illustrated in FIG. 2B.

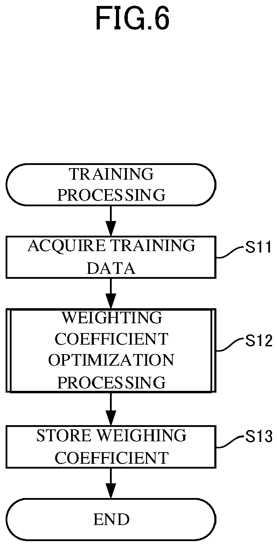

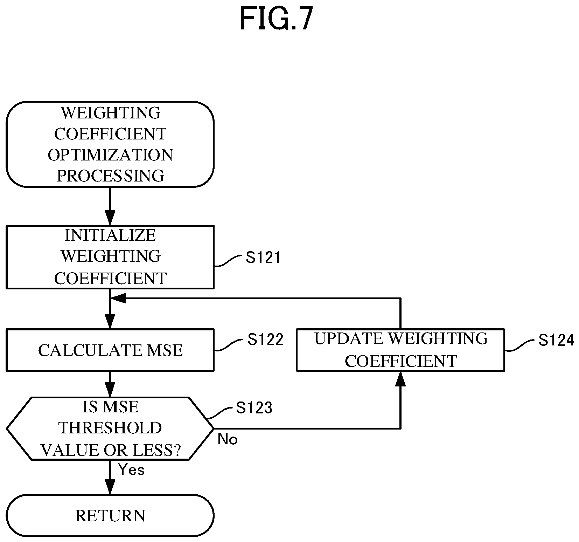

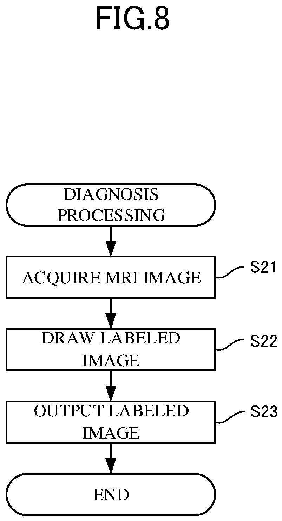

Returning to FIG. 2A again, the controller 150 includes a processor, such as a central processing unit (CPU), and performs control of each unit of the image diagnosis apparatus 100. The controller 150, by executing the programs stored in the storage 140, executes each of training processing in FIG. 6, weighting coefficient optimization processing in FIG. 7, and diagnosis processing in FIG. 8. The controller 150 functionally includes a trainer 151, an acquirer 152, a drawer 153, and an outputter 154.

The trainer 151 generates a trained model by machine learning with reference to training data stored in the training data storage 141 and stores the generated trained model in the trained model storage 142. The trainer 151 generates a trained model by performing supervised learning, using a plurality of data sets included in the training data as teacher data.

The trainer 151, using the plurality of data sets included in the training data as teacher data, adjusts weighting coefficients that indicate connection states between the layers in the neural network. When specifically described, the trainer 151 compares the pixel values of respective pixels in a labeled image, the labeled image being output from the output layer as output data by inputting the pixel values of respective pixels in the MRI image to the input layer as input data, with the pixel values of respective pixels in a labeled image in the teacher data. The trainer 151 optimizes the weighting coefficients in such a manner that differences between the pixel values of the respective pixels in the labeled image output from the output layer and the pixel values of the respective pixels in the labeled image in the teacher data are made as small as possible. For the optimization of the weighting coefficients, for example, an error back-propagation method is used.

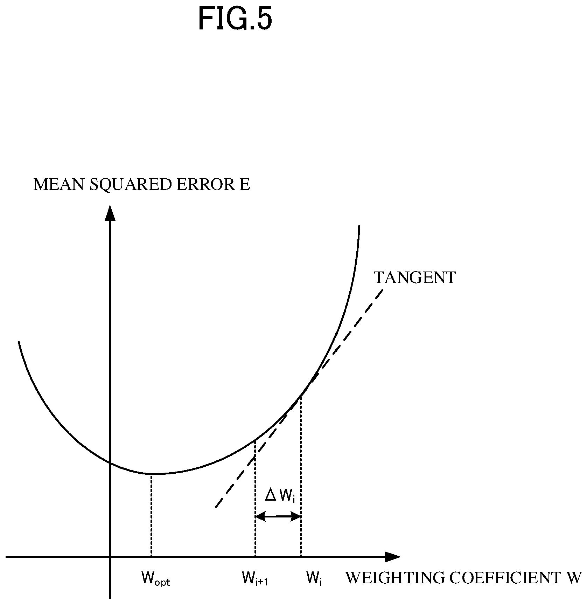

With reference to FIG. 5, a process for optimizing weighting coefficients, using the error back-propagation method is described below. First, based on the pixel values of the respective pixels in a labeled image (estimated values of output data) output from the neurons for output by inputting the pixel values of the respective pixels in the MRI image (input data) to the neurons for input in the input layer and the pixel values of the respective pixels in a labeled image generated manually (set values of output data), a mean squared error (MSE) between the estimated values and the set values of the output data is calculated. The MSE is expressed by the following equation (1). Note, however, that i = 1 to m and j = 1 to n.

$$MSE = \Sigma(I(i, j) - O(i, j))^2/(m \times n) \ldots (1)$$

Next, by minimizing the MSE, the weighting coefficients W of the connections between the layers in the neural network are optimized. Optimized weighting coefficients when the MSE has a minimum value are denoted by $W_{opt}$. To minimize the MSE, it is only required to use, for example, a gradient descent method. The gradient descent method is a method of decreasing a value of the MSE by repeating a process of calculating a gradient of the MSE and updating the weighting coefficients W in the opposite direction to a direction of change in the magnitude of the gradient and thereby acquiring optimized weighting coefficients $W_{opt}$. When current weighting coefficients, updated weighting coefficients, and change amounts of weighting coefficients are denoted by $W_i$, $W_{i+1}$, and $\Delta W_i$, respectively, the current weighting coefficients, the updated weighting coefficients, and the change amounts of the weighting coefficients satisfy the following equation (2).

$$W_{i+1} = W_i + \Delta W_i \ldots (2)$$

Since the gradient of the MSE is represented by partial derivatives ($\partial E/\partial W$) that are obtained by differentiating the MSE with respect to each of the weighting coefficients W, the change amounts $\Delta W_i$ of the weighting coefficients are expressed by the following equation (3).

$$\Delta W_i = -\eta \times (\partial E/\partial W_i) \ldots (3)$$

In the equation (3), $\eta$ is a coefficient satisfying $0 < \eta < 1$. By substituting the equation (3) into the equation (2), updated weighting coefficients $W_{i+1}$ can be obtained.
The foregoing is the process for optimizing the weighting coefficients, using the error back-propagation method.
Returning to FIG. 2A, the acquirer 152 acquires image data of the subject that are transmitted from the MRI apparatus 200 and stores the acquired image data in the image data storage 143 in association with the subject ID. The acquisition of image data by the acquirer 152 includes both reception of image data from an external apparatus and retrieval of data stored in the storage 140.
The drawer 153 draws a labeled image corresponding to an image data of the subject acquired by the acquirer 152, based on the trained model that is generated by the trainer 151 and stored in the trained model storage 142.
The outputter 154 outputs a labeled image drawn by the drawer 153 to the outside. The outputter 154, for example, transmits image data representing a labeled image obtained by the drawer 153 to the display 120 and causes the display 120 to display the labeled image. The outputter 154 may color-codes each class in the labeled image to facilitate understanding by the user and output the color-coded labeled image. In addition, the outputter 154 may cause the display 120 to display the labeled image drawn by the drawer 153 and the original MRI image side by side.
The foregoing is the configuration of the image diagnosis apparatus 100.

Training Processing

With reference to a flowchart in FIG. 6, a flow of the training processing that the controller 150 of the image diagnosis apparatus 100 executes is described below. The training processing is processing of generating, based on training data including an MRI image and a labeled image generated manually, a trained model by which a labeled image indicating existence or nonexistence of a brain tumor is drawn. The training processing is started at a time point when after training data are generated and stored in the training data storage 141, the user, by operating the operation acceptor 110, instructs start of the training processing.
First, the acquirer 152 acquires training data stored in the training data storage 141 (step S11).
Next, the trainer 151 executes weighting coefficient optimization processing of optimizing weighting coefficients included in the training data (step S12). With reference to FIG. 7, a flow of the weighting coefficient optimization processing is described below.
First, the trainer 151 initializes each of weighting coefficients between all the neurons in the respective layers of the neural network (step S121). An initial value of each weighting coefficient between neurons is only required to be given by, for example, a random number from -0.1 to +0.1.
Next, the trainer 151 outputs the pixel values of respective pixels in a labeled image (predicted values of output data) that is output from neurons for output in the output layer by inputting the pixel values of respective pixels in the MRI image (input data) to neurons for input in the input layer and calculates an MSE between the predicted values of the output data and the pixel values of respective pixels in the labeled image that a medical specialist labels manually (set values of output data) (step S122).

Next, the trainer 151 determines whether or not the MSE is less than or equal to a threshold value (step S123). When the MSE is determined to be less than or equal to the threshold value (step S123; Yes), the trainer 151 returns the weighting coefficient optimization processing to the training processing. In contrast, when the MSE is determined not to be less than or equal to the threshold value (step S123; No), the trainer 151 updates the values of the respective weighting coefficients $W_i$ to $W_{i+1}$ by adding change amounts $\Delta W_i$ calculated using the equation (3) to the values of the respective weighting coefficients $W_i$ (step S124) and returns the process to step S122.

The foregoing is the flow of the weighting coefficient optimization processing.

Returning to FIG. 6, the trainer 151 stores the respective updated weighting coefficients $W_{i+1}$ that are finally obtained, in the trained model storage 142 as optimized weighting coefficients $W_{opt}$ (step S13) and terminates the process.

The foregoing is the flow of the training processing.

(Diagnosis Processing)

With reference to a flowchart in FIG. 8, a flow of the diagnosis processing that the controller 150 of the image diagnosis apparatus 100 executes is described below. The diagnosis processing is processing of drawing a labeled image indicating existence or nonexistence of a brain tumor, based on an MRI image of the subject. The diagnosis processing is started at a time point when after a trained model is generated by the training processing, the user, by operating the operation acceptor 110, instructs start of the diagnosis processing.

First, the acquirer 152 acquires an MRI image corresponding to the subject ID of the subject to be diagnosed from the image data storage 143 (step S21).

Next, the drawer 153 retrieves the trained model stored in the trained model storage 142 and draws a labeled image corresponding to the MRI image from the MRI image acquired in the processing in step S21, using the trained model (step S22). Specifically, using the optimized weighting coefficient $W_{opt}$ between all the neurons in the respective layers of the neural network, calculation processing is successively performed from the intermediate layers to the output layer, and the pixel values of respective pixels in the labeled image are estimated.

Next, the outputter 154 outputs image data relating to the labeled image drawn in the processing in step S22 to the outside (step S23). The outputter 154, for example, transmits the labeled image drawn in the processing in step S22 to the display 120 and causes the display 120 to display the labeled image that is color-coded with respect to each class.

The foregoing is the flow of the diagnosis processing.

As described in the foregoing, the image diagnosis apparatus 100 according to the embodiment includes the acquirer 152 to acquire an MRI image of the brain of a subject and the drawer 153 to, based on the MRI image acquired by the acquirer 152, draw a labeled image that is partitioned based on classes each of which indicates one of the tumor area, the cavity area, the soft tissue area, the skull area, and the background area. Thus, even when the user is not a person of experience in the image diagnosis, the user is able to diagnose a brain tumor of the subject easily and accurately.

In addition, the image diagnosis apparatus 100 according to the embodiment includes the trainer 151 to generate a model for estimating the pixel values of respective pixels in a labeled image from the pixel values of respective pixel in an MRI image by machine learning. Thus, a brain tumor of the subject can be diagnosed more accurately, using a trained model generated by the machine learning.

The present disclosure is not limited to the above-described embodiment, and modified examples described below can also be embodied.

Modified Examples

Although in the above-described embodiment, an image of a cross section of the brain of a subject obtained by slicing the brain in the transverse plane was used, the present disclosure is not limited thereto. For example, an image of a cross section of the brain of the subject obtained by slicing the brain in the frontal plane or the sagittal plane may be used.

Although in the above-described embodiment, an MRI image of a cross section of the brain of the subject obtained by slicing the brain in the transverse plane was used as a tomographic image of the brain of the subject, the present disclosure is not limited thereto. For example, a medical tomographic image may be a CT image, an X-ray image, or an ultrasonic image that is obtained by capturing the transverse plane of the brain of the subject. In this case, it is only required that the MRI apparatus 200 is replaced by another medical image capturing apparatus, such as a CT apparatus, an X-ray image capturing apparatus, and an ultrasonic tomographic image capturing apparatus and the medical image capturing apparatus is connected to the image diagnosis apparatus 100 in a communicable manner.

Although in the above-described embodiment, a labeled image was partitioned into five classes, namely the tumor area, the cavity area, the soft tissue area, the skull area, and the background area, the present disclosure is not limited thereto. For example, the number of classes of the labeled image may be set to four or less. Specifically, a labeled image may be separated into classes consisting of a tumor area, a cavity area, a normal tissue area including soft tissue and the skull, and a background area or may be separated into classes consisting of a tumor area, a normal tissue area including a cavity, soft tissue, and the skull, and a background area. Alternatively, another class may be added to the above-described five classes, and the soft tissue area may be separated into classes consisting of a cerebrum area, a cerebellum area, and a brainstem area, and the cerebrum area may be further separated into classes consisting of a cerebral cortex area, a gray matter area, and a white matter area.

Although in the above-described embodiment, both the MRI image and the labeled image were grayscale images with 256 gradations (8 bits), the present disclosure is not limited thereto. The MRI image and the labeled image may be, for example, grayscale image with 16 bits per pixel or a color image. The color image is, for example, a 24-bit image where each of red-green-blue (RGB) elements at a pixel is represented by 8 bits. In addition, a labeled image that is a color image may be drawn from an MRI image that is a grayscale image, using a trained model.

Although in the above-described embodiment, the trainer 151 performed machine learning, using a neural network, the present disclosure is not limited thereto. A machine learning method other than a neural network, such as regression analysis using a support vector machine (support vector regression), may be used. In addition, since a training model defines a relationship between input data and output data, a training model may be constructed using a method other than the machine learning as long as the method can acquire output data from an output layer when data are supplied to an input layer.

Although in the above-described embodiment, the image diagnosis apparatus 100 included the function of the trainer 151, the present disclosure is not limited thereto. For example, an external apparatus that is separate from the image diagnosis apparatus 100 may include the function of the trainer 151. In this case, it is only required that the external apparatus generates a trained model by learning a relationship between an MRI image and a labeled image, based on training data and the image diagnosis apparatus 100 acquires the trained model generated by the external apparatus through communication performed by the communicator 130.

Although in the above-described embodiment, optimized weighting coefficients obtained through the training processing using training data were stored in the trained model storage 142 without change, the present disclosure is not limited thereto. For example, it may be configured such that after the training processing is performed using a portion of training data, validity of optimized weighting coefficients is evaluated using the remainder of the training data and when the weighting coefficients are evaluated to be valid weighting coefficients, the weighting coefficients are stored in the trained model storage 142.

Although in the above-described embodiment, a labeled image was drawn using a trained model, based on an MRI image of a subject that was captured by the MRI apparatus 200, the present disclosure is not limited thereto. For example, it may be configured such that by capturing an MRI image captured by a medical image capturing apparatus, using a computer with camera while the MRI image is displayed on a monitor, the captured image is taken into the computer with camera and a labeled image is drawn in the computer by performing diagnosis processing using a trained model. The computer with camera is, for example, a smartphone or a tablet terminal, and it is only required that an application that executes diagnosis processing based on a taken-in image is installed in the computer with camera in advance.

Although in the above-described embodiment, the outputter 154 caused the display 120 to display a labeled image that was color-coded with respect to each class, the present disclosure is not limited thereto. For example, it may be configured such that the controller 150 of the image diagnosis apparatus 100 includes a determiner to determine whether or not a brain tumor exists, based on a labeled image drawn by the drawer 153 and a determination result by the determiner is output from the outputter 154. The determiner is only required to determine that a brain tumor exists in the subject when pixels having pixel values equivalent to a tumor area exist in the labeled image. When it is determined by the determiner that a brain tumor exists in the subject, the outputter 154 may notify the user of a warning to the effect that a brain tumor exists in the subject. As the warning to the user, the display 120 may be caused to display a warning screen, or a warning sound may be generated from a speaker of the image diagnosis apparatus 100. In addition, the outputter 154 may cause the display 120 to display the labeled image and the original MRI image side by side, as well as displaying a site corresponding to a tumor area in the MRI image by magnifying or highlighting the site.

Although in the above-described embodiment, the brain was targeted as a region to be diagnosed and the image diagnosis apparatus 100 diagnosed existence or nonexistence of a brain tumor in the brain of the subject, the present disclosure is not limited thereto. For example, existence or nonexistence of a tumor that develops in other internal organs, such as the stomach, the lungs, the liver, the spleen, the large intestine, the bladder, the prostate, the mamma, and the womb of the subject, may be diagnosed. In this case, a labeled image is only required to be partitioned into, for example, five classes, namely a tumor area, a cavity area, a soft tissue area, a bone area, and a background area. In addition, a diagnosis target of the image diagnosis apparatus 100 is not limited to a tumor having developed in the internal organs and may be a lesion having some graphic characteristics, such as inflammation, scarring, and fiberization.

Although in the above-described embodiment, various types of data were stored in the storage 140 of the image diagnosis apparatus 100, the present disclosure is not limited thereto. For example, all or some of various types of data may be stored in an external control apparatus or an external computer via a communication network.

Although in the above-described embodiment, the image diagnosis apparatus 100 operated based on the programs stored in the storage 140, the present disclosure is not limited thereto. For example, a functional configuration that is achieved by programs may be achieved by hardware.

Although in the above-described embodiment, the image diagnosis apparatus 100 was, for example, a general-purpose computer, the present disclosure is not limited thereto. For example, the image diagnosis apparatus 100 may be achieved by a computer installed on a cloud.

Although in the above-described embodiment, the processing that the image diagnosis apparatus 100 executes was achieved by execution of programs stored in the storage 140 by an apparatus including the above-described physical configuration, the present disclosure may be achieved as programs or may be achieved as a non-transitory recording medium in which the programs are recorded.

In addition, an apparatus that executes the above-described processing operation may be configured by storing and distributing programs for causing the above-described processing operation to be executed in a non-transitory computer-readable recording medium, such as a flexible disk, a compact disk read-only memory (CD-ROM), a digital versatile disk (DVD), and a magneto-optical disk (MO), and installing the programs in a computer.

The above-described embodiment is only an exemplification, and the present disclosure is not limited to the embodiment and various embodiments can be embodied without departing from the scope of the present disclosure described in the claims. The constituent elements described in the embodiment and modified examples can be freely combined. In addition, an invention equivalent to the invention described in the claims is also included in the present disclosure.

The present disclosure is specifically described below by way of Examples. However, the present disclosure is not limited to Examples.

Examples

In Example, machine learning was performed using MRI images of brain tumor patients, and existence or nonexistence of a brain tumor in the brains of patients was determined using a trained model generated by the machine learning.

First, MRI images were acquired from 51 patients who had a metastatic brain tumor diagnosis for the first time. The patients had been confirmed that carcinomatous meningitis, glioblastoma, or bone metastasis had not concurred with metastatic brain tumor. The MRI images were T1-weighted images (T1WI) that were captured while the Gadolinium-based contrast agent (Gd-based contrast agent) was administered. Slice pitch was 3 to 5 mm, and the number of slices for each patient was 60 to 100. All the slices were used with respect to 10 cases, and only slices including an imaged lesion were used with respect to other 41 cases. The total number of images used was 1507. In order to perform generation and evaluation of a training model, the MRI images acquired from 51 patients were distributed in a random manner at a ratio of 80% for images for training and 20% for images for test. As a result, the number of the images for training and the number of the images for test were 1206 and 301, respectively.

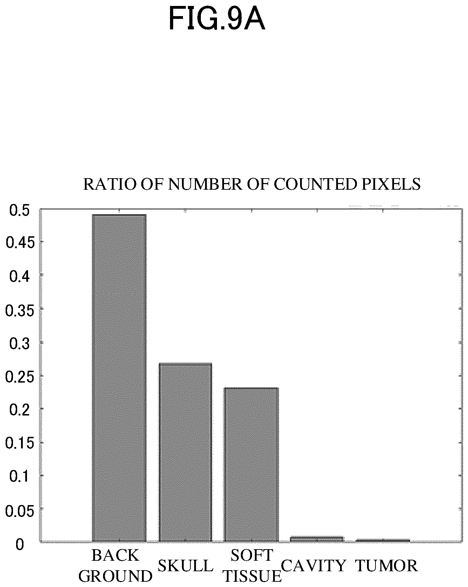

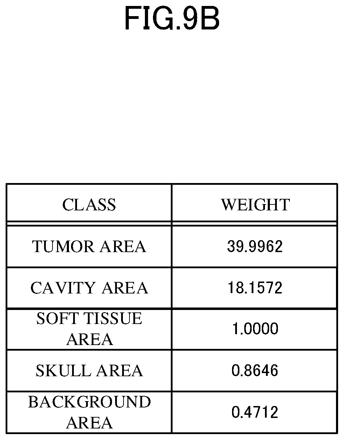

Next, labeled images each of which is obtained by segmenting all the pixels in the image into classes were generated from the images for training. A medical specialist repeated manually generating a labeled image with respect to each MRI image. Each of classes set in a labeled image is one of the tumor area, the cavity area, the soft tissue area, the skull area, and the background area. For setting of classes, Image Labeler was used. In order to set a weight for each class, the number of pixels of each class in each of the labeled images was counted. In consideration of the number of counted pixels of each label illustrated in FIG. 9A, a weight of each class was set as illustrated in FIG. 9B.

Next, by causing supervised learning to be performed based on training data including images for training and labeled images, a trained model was generated. For the generation of the trained model, MATLAB (registered trademark) was used. Subsequently, a labeled image was caused to be drawn from each image for test, using the generated trained model, and whether or not a brain tumor was able to be detected in each of the drawn labeled images was confirmed.

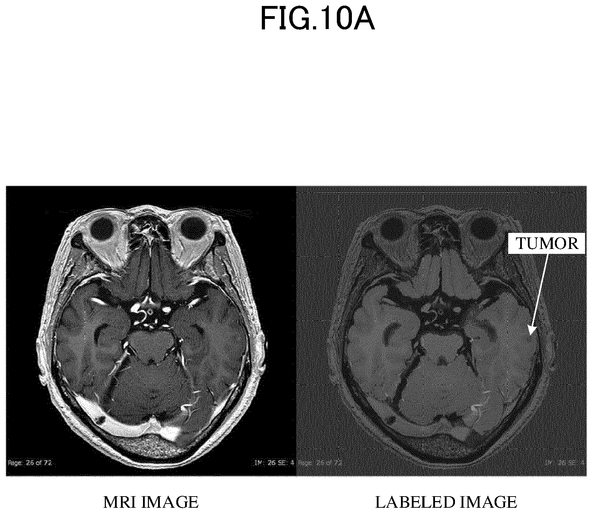

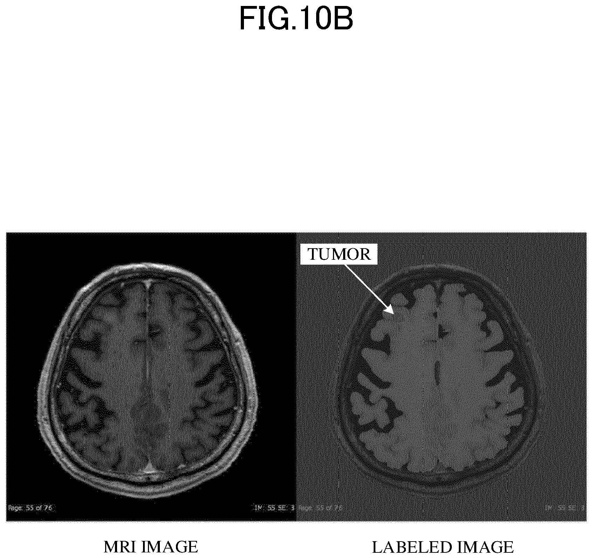

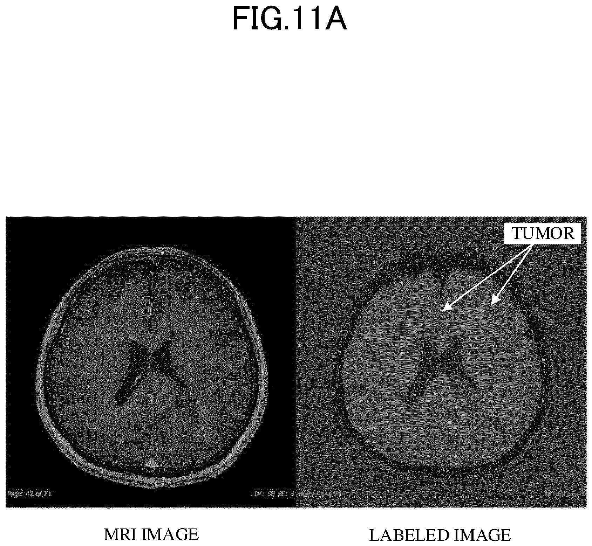

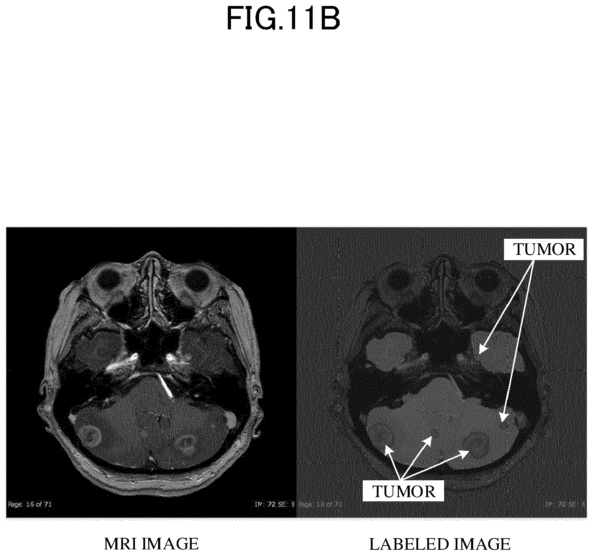

A test result is described below. As a result of drawing labeled images from images for test, using the trained model, the test succeeded in detecting a small single lesion and a lesion having low pixel values, as illustrated in FIGS. 10A and 10B. In addition, as illustrated in FIGS. 11A and 11B, the test also succeeded in detecting an extremely small lesion and multiple lesions. As a result of calculation of 301 test images, the number of tumors was 326, the number of false negatives was 13, the number of false positives was 76, and the number of false positives in the skull among the false positives was 64. A diagnosis rate that indicates a ratio of persons who were not misdiagnosed to persons who were diagnosed to have a tumor in diagnosis using the trained model was 96%. In addition, the test succeeded in suppressing the number of false positives to a low level in such a manner that the number of false positives per image was 0.25 and was 0.21 on the inside of the skull.

This application claims the benefit of Japanese Patent Application No. 2021-166163, filed on October 8, 2021, the entire disclosure of which is incorporated by reference herein.

Industrial Applicability

The image diagnosis apparatus, the method for operating the image diagnosis apparatus, and the program of the present disclosure assist simple and accurate diagnosis of existence or nonexistence of a lesion in a region to be diagnosed and are therefore useful.

Reference Signs List

1        Image diagnosis system
100     Image diagnosis apparatus
110     Operation acceptor
120     Display
130     Communicator
140     Storage
141     Training data storage
142     Trained model storage
143     Image data storage
150     Controller
151     Trainer
152     Acquirer
153     Drawer
154     Outputter
200     MRI apparatus

**Claims**

1.  An image diagnosis apparatus, comprising:

    an acquirer to acquire a tomographic image including a region to be diagnosed of a subject; and
    a drawer to draw, based on a tomographic image acquired by the acquirer, a labeled image including the region to be diagnosed, the labeled image being partitioned according to classes each of which indicates one of a lesion area, a normal tissue area, and a background area.

2.  The image diagnosis apparatus according to claim 1, wherein the normal tissue area is partitioned into a cavity area, a soft tissue area, and a bone area.

3.  The image diagnosis apparatus according to claim 1 or 2, wherein

    the lesion area is a tumor area in a brain,
    the tomographic image is an image of a cross section of a brain of a subject, the cross section being obtained by slicing the brain in a transverse plane direction at a plurality of points, and
    the drawer draws a labeled image corresponding to each tomographic image acquired by the acquirer.

4.  The image diagnosis apparatus according to claim 3, wherein the tumor area is an area where a metastatic brain tumor has developed.

5.  The image diagnosis apparatus according to any one of claims 1 to 4, wherein

    the labeled image has a unique pixel value with respect to each class, and
    the drawer estimates, based on a model that, with respect to input of a pixel value of each pixel in a tomographic image, outputs a pixel value of each pixel in a labeled image, a pixel value of each pixel in a labeled image from a tomographic image acquired by the acquirer.

6.  The image diagnosis apparatus according to claim 5, wherein

    the image diagnosis apparatus further includes a trainer to generate the model by machine learning, and
    the drawer estimates, based on the model generated by the trainer, a pixel value of each pixel in a labeled image from a pixel value of each pixel in a tomographic image acquired by the acquirer.

7.  The image diagnosis apparatus according to claim 6, wherein the trainer generates the model, using training data that include a pixel value of each pixel in a tomographic image as input data and a pixel value of each pixel in a labeled image as output data, the labeled image being generated based on the tomographic image and having a weight of each class adjusted based on a number of counted pixels of the class.

8.  The image diagnosis apparatus according to claim 6 or 7, wherein the trainer generates the model, using training data generated based on a plurality of tomographic images that has cross sections obtained by slicing a brain of each of a plurality of subjects in a transverse plane direction at a plurality of points.

9.  The image diagnosis apparatus according to any one of claims 1 to 8, wherein the image diagnosis apparatus further includes an outputter to color-code a labeled image with respect to each class, the labeled image being drawn by the drawer.

10. A method for operating an image diagnosis apparatus including an acquirer and a drawer, the method comprising:

acquiring, by the acquirer, a tomographic image including a region to be diagnosed of a subject; and

drawing, by the drawer, based on a tomographic image acquired by the acquirer, a labeled image including the region to be diagnosed, the labeled image being partitioned according to classes each of which indicates one of a lesion area, a normal tissue area, and a background area.

11. A program for causing a computer to function as:

acquisition means for acquiring a tomographic image including a region to be diagnosed of a subject; and

drawing means for drawing, based on a tomographic image acquired by the acquisition means, a labeled image including the region to be diagnosed, the labeled image being partitioned according to classes each of which indicates one of a lesion area, a normal tissue area, and a background area.

# FIG.1

# FIG.2A

IMAGE DIAGNOSIS APPARATUS

<u>100</u>

110 — OPERATION ACCEPTOR

120 — DISPLAY

130 — COMMUNICATOR

140 — STORAGE
141 — TRAINING DATA STORAGE
142 — TRAINED MODEL STORAGE
143 — IMAGE DATA STORAGE

150 — CONTROLLER
151 — TRAINER
152 — ACQUIRER
153 — DRAWER
154 — OUTPUTTER

# FIG.2B

IMAGE DATA STORAGE 143

| SUBJECT ID | IMAGE DATA |
|:----------:|:----------:|
| 1001 | M1 |
| 1002 | M2 |
| ⋮ | ⋮ |

# FIG.3

IMAGE FOR TRAINING                    LABELED IMAGE

CAVITY AREA

SKULL AREA

TUMOR AREA

BACKGROUND AREA          SOFT TISSUE AREA

SET OF TRAINING DATA

# FIG.4

# FIG.5

# FIG.6

```
┌─────────────────────────┐
│      TRAINING           │
│     PROCESSING          │
└─────────────────────────┘
            │
            ▼
┌─────────────────────────┐
│   ACQUIRE TRAINING      │── S11
│        DATA             │
└─────────────────────────┘
            │
            ▼
┌─────────────────────────┐
│      WEIGHTING          │
│     COEFFICIENT         │── S12
│    OPTIMIZATION         │
│     PROCESSING          │
└─────────────────────────┘
            │
            ▼
┌─────────────────────────┐
│   STORE WEIGHING        │── S13
│    COEFFICIENT          │
└─────────────────────────┘
            │
            ▼
┌─────────────────────────┐
│         END             │
└─────────────────────────┘
```

# FIG.7

```
┌─────────────────────┐
│     WEIGHTING       │
│    COEFFICIENT      │
│   OPTIMIZATION      │
│    PROCESSING       │
└─────────────────────┘
           │
           ▼
┌─────────────────────┐
│     INITIALIZE      │── S121
│     WEIGHTING       │
│    COEFFICIENT      │
└─────────────────────┘
           │
           ▼
┌─────────────────────┐              ┌─────────────────────┐
│   CALCULATE MSE     │── S122       │ UPDATE WEIGHTING    │── S124
└─────────────────────┘              │    COEFFICIENT      │
           │                         └─────────────────────┘
           ▼                                    ▲
     ╱─────────────╲                            │
    ╱   IS MSE      ╲── S123                     │
   ╱  THRESHOLD      ╲ ──────── No ──────────────┘
    ╲ VALUE OR LESS? ╱
     ╲──────┬──────╱
         Yes│
            ▼
┌─────────────────────┐
│      RETURN         │
└─────────────────────┘
```

# FIG.8

```
┌─────────────────────┐
│     DIAGNOSIS       │
│    PROCESSING       │
└─────────────────────┘
          │
          ▼
┌─────────────────────┐
│  ACQUIRE MRI IMAGE  │───  S21
└─────────────────────┘
          │
          ▼
┌─────────────────────┐
│   DRAW LABELED      │───  S22
│      IMAGE          │
└─────────────────────┘
          │
          ▼
┌─────────────────────┐
│  OUTPUT LABELED     │───  S23
│      IMAGE          │
└─────────────────────┘
          │
          ▼
┌─────────────────────┐
│        END          │
└─────────────────────┘
```

# FIG.9A

RATIO OF NUMBER OF COUNTED PIXELS

# FIG.9B

| CLASS | WEIGHT |
|---|---|
| TUMOR AREA | 39.9962 |
| CAVITY AREA | 18.1572 |
| SOFT TISSUE AREA | 1.0000 |
| SKULL AREA | 0.8646 |
| BACKGROUND AREA | 0.4712 |

# FIG.10A

MRI IMAGE                    LABELED IMAGE

# FIG.10B

TUMOR

MRI IMAGE                  LABELED IMAGE

# FIG.11A

MRI IMAGE                    LABELED IMAGE

# FIG.11B

MRI IMAGE                    LABELED IMAGE

## INTERNATIONAL SEARCH REPORT

| International application No. |
| --- |
| **PCT/JP2022/037568** |

| | |
| --- | --- |
| **A. CLASSIFICATION OF SUBJECT MATTER** | |
| *A61B 5/055*(2006.01)i; *G06T 7/00*(2017.01)i<br>FI: A61B5/055 380; G06T7/00 612 | |
| According to International Patent Classification (IPC) or to both national classification and IPC | |

| **B. FIELDS SEARCHED** |
| --- |
| Minimum documentation searched (classification system followed by classification symbols)<br>    A61B5/055; G06T7/00 |
| Documentation searched other than minimum documentation to the extent that such documents are included in the fields searched<br>        Published examined utility model applications of Japan 1922-1996<br>        Published unexamined utility model applications of Japan 1971-2022<br>        Registered utility model specifications of Japan 1996-2022<br>        Published registered utility model applications of Japan 1994-2022 |
| Electronic data base consulted during the international search (name of data base and, where practicable, search terms used) |

| **C. DOCUMENTS CONSIDERED TO BE RELEVANT** | | |
| --- | --- | --- |
| Category* | Citation of document, with indication, where appropriate, of the relevant passages | Relevant to claim No. |
| X | DESPOTOVIC, Ivana et al. MRI Segmentation of the Human Brain: Challenges, Methods, and Applications. Computation and Mathematical Methods in Medicine, 2015, vol. 2015, pp. 1-23<br>    1. Introduction to 6.Discussion and Conclusions | 1-11 |

☐ Further documents are listed in the continuation of Box C.     ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **21 October 2022** | **20 December 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018515164 A
- JP 2021166163 A